# EUROPEAN PATENT APPLICATION

(11) **EP 2 698 148 A1**
(43) Date of publication of application: **19.02.2014**
(21) Application number: 13180714.1
(22) Date of filing: 16.08.2013
(51) Int. Cl.: A61K 9/00, A61K 31/138, A61K 31/4985, A61K 9/68

(54) **Chewing Gum Formulations Comprising Dapoxetine and Tadalafil**

(30) Priority: 17.08.2012 TR 201209599; 31.10.2012 TR 201212488; 07.11.2012 TR 201212850; 07.02.2013 TR 201301536; 08.02.2013 TR 201301559; 30.05.2013 TR 201306476
(71) Applicant: Sanovel Ilac Sanayi ve Ticaret A.S., 34460 Istanbul (TR)
(72) Inventor: Türkyilmaz, Ali, 34460 Istanbul (TR); Yelken, Gülay, 34460 Istanbul (TR)
(74) Representative: Sevinç, Erkan

(57) **Abstract**

The present invention relates to chewing gum formulations of dapoxetine or a pharmaceutically acceptable salt thereof and tadalafil or a pharmaceutically acceptable salt thereof. The present invention further relates to a process for preparing such a formulation and to the use thereof in the treatment of erectile dysfunction.

## Description

### Field of Invention

The present invention relates to a formulation comprising a combination of dapoxetine or a pharmaceutically acceptable salt thereof and tadalafil or a pharmaceutically acceptable salt thereof. The present invention particularly relates to a chewing gum formulation comprising dapoxetine and tadalafil.

### Background of Invention

Selective serotonin reuptake inhibitors (SSRI) are used in the long-term prophylaxis of many types of depression, including the endogenous type, recurrent depression, and in the treatment of obsessive-compulsive disorders, panic attack, social phobias, and the bulimia nervosa disease. Dapoxetine, which was first disclosed in the European patent publication EP 0288188 B1 is a selective serotonin reuptake inhibitor. Dapoxetine is used for the treatment of depression and premature ejaculation and has the chemical structure shown in Formula I. Additionally, dapoxetine was approved in Switzerland and in Finland for use in the treatment of premature ejaculation.

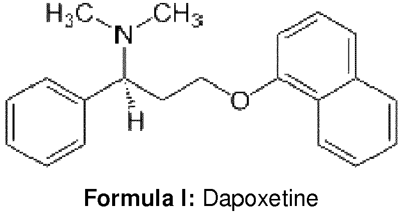

Following oral administration, dapoxetine is rapidly absorbed and rapidly enters the blood circulation by almost completely binding to plasma proteins. Therefore, it achieves the peak plasma concentration (Cmax) in 1 hour following oral administration. Orally-administered tablets of dapoxetine are commercially available under the name Priligy^{®}, comprising 30 mg or 60 mg dapoxetine hydrochloride per tablet, as well as excipients including lactose monohydrate, microcrystalline cellulose, croscarmellose sodium, colloidal anhydrous silica, magnesium stearate, hypromellose, titanium dioxide (E171), triacetin, black iron oxide (E172) and yellow iron oxide.

The most frequently encountered problem in oral dapoxetine formulations is its bitter taste. The tablets have typically been coated with coating agents, and mixtures of sweeteners or cation exchange resins have been used for masking the bitter taste.

On the other hand, phosphodiesterase type 5 inhibitors (PDE5 inhibitor) are used in the treatment of erectile dysfunction (ED). PDE5 inhibitors block the phosphodiesterase enzyme in a selective and efficient manner, thus increasing the level of cyclic guanosine monophosphate (cGMP) in the corpus cavernosum smooth muscle cells. The most frequently used PDE5 inhibitors are avanafil, lodenafil, mirodenafil, sildenafil, tadalafil, vardenafil, and udenafil. Tadalafil is a PDE5 inhibitor used in the treatment of ED and PAH. It has a longer half life as compared to other PDE5 inhibitors (mean, 17,5 hours). The chemical designation of tadalafil is (6R-trans)-6-(1,3-benzodioxol-5-yl)-2,3,6,7,12,12a-hexahydro-2-methyl-pyrazino [1',2':1,6] pyrido[3,4-b]indole-1,4-dione, with the chemical structure illustrated below in Formula II.

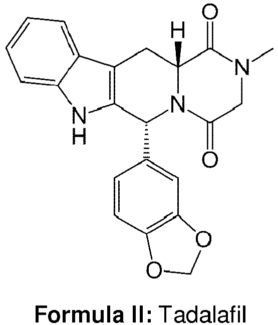

Formulations comprising a combination of selective serotonin reuptake inhibitors with PDE5 inhibitors are known in the prior art. The patent publication WO03000343, for example, discloses the use of a formulation comprising a combination of phosphodiesterase inhibitors and dapoxetine. It was reported in a study that dapoxetine, a selective serotonin reuptake inhibitor, and PDE5 inhibitors do not show a pharmacokinetic interaction and therefore can be used in the treatment of premature ejaculation.

The formulations comprising a combination of selective serotonin reuptake inhibitors with PDE5 inhibitors are known in the prior art. It was reported in a study that dapoxetine, a selective serotonin reuptake inhibitor, and PDE5 inhibitors do not show a pharmacokinetic interaction and therefore can be used in the treatment of premature ejaculation. Additionally, although that chewing gum formulations comprising tadalafil were disclosed in the prior art, no chewing gum formulations are present in which dapoxetine and tadalafil are used together.

Various formulations and methods for preparing chewing gum formulations are already known. However, concerning oral administration, chewing gum formulations have become an issue with increasing importance in terms of patient compliance as compared to the conventional solid dosage forms such as capsules and tablets. Probably the most important advantage of chewing gum dosage forms is that the active agents comprised therein dissolve at a slow rate in the oral cavity for a relatively long time period. Thus, a patient can be treated without becoming exposed to a large amount of drug, as it the case in tablets, and this point is very important for those drugs such as tadalafil which lead to vasodilation. This is because an abrupt dose increase, may increase the heart rate so as to possibly result in heart attack. However, since an active agent in a chewing gum formulation is released slowly, chewing gum formulations do not cause said side effects. Additionally, since tadalafil which is poorly soluble in water is similarly poorly dissolved in gastric juice, its absorption in the stomach is low. Chewing gum formulations provide for a more efficient absorption of the drug in the buccal mucosa, and this may reduce the first pass effect and thus the efficiency of the drug can be enhanced. Since this dosage form stays away from the hepatic first-pass metabolism, it increases the clinic effects of some drugs by increasing the bioavailability and decreasing the side-effects thereof.

Developing chewing gum formulations is known to be difficult for several different reasons. First, the bitter taste of the active agents comprised in a chewing gum formulation may be problematic. Moreover, these compositions should be flexible and not too rigid to provide an easy chewing. These compositions must have a sufficient viscosity to be turned into a chewing gum form.

In order to fulfill all these requirements described above, a special drug formulation is needed and therefore the excipients should be selected carefully. The selected excipients, however, may give formulations with decreased bioavailability as compared to equivalent conventional dosage forms. For this reason, the excipients should be selected very carefully.

Based on the above reasons, it would be very desirable to have a chewing gum formulation of dapoxetine and tadalafil. Other advantages and embodiments of the present invention will be clarified in the following description.

### Detailed Description of Invention

The main object of the present invention is to provide a chewing gum formulation of a combination of dapoxetine or a pharmaceutically acceptable salt thereof and tadalafil or a pharmaceutically acceptable salt thereof by making use of suitable excipients, said formulation being useful in the treatment of erectile dysfunction and the related symptoms thereof, overcoming the problems given above, as well as being highly effective, having an adequate viscosity and a pleasant taste. Another object of the present invention, in turn, is to develop a chewing gum base having an adequate plasticity.

Thus, a chewing gum formulation of dapoxetine and tadalafil is obtained according to the present invention, providing a slow release in the oral cavity and therefore having increased bioavailability. Accordingly, the present invention provides a chewing gum formulation comprising dapoxetine or a pharmaceutically acceptable salt thereof and tadalafil or a pharmaceutically acceptable salt thereof.

Chewing gum formulations comprise a water-soluble part and a water-insoluble part. While the water-insoluble part is rather composed by a chewing gum base, elastomers, and other fillers, the water-soluble part is composed by emulsifying agents, plasticizers, sweeteners, flavors, and colorants. Since chewing gum formulations stay for a long period of time in the mouth, it is important to provide these formulations with a pleasant taste. Therefore, the majority of the water-soluble part is composed of sweeteners. In this context, it was found that the use of sucralose in defined proportions improved the taste of the chewing gum formulation of dapoxetine and tadalafil in a surprising manner. Since sucralose is a synthetic sweetener, it has no caloric value and does not cause tooth decays. As it does not influence the blood insulin level, it can also be safely administrated to the diabetics, and it is advantageous in terms of patient compliance. Additionally, since the sweetening power of sucralose is around 600 times higher than that of sucrose, satisfactory results were obtained even at low amounts of sucralose. Also since sucralose is a free-flowing crystalline powder, it facilitates the compression procedures. Using sucralose in an amount of 0.10 to 2.00% of the formulation according to the present invention was observed to be effective in preventing the bitter taste.

It was further observed that using defined amounts of glycerin as an emulsifying agent in the chewing gum formulations according to the present invention comprising dapoxetine or a pharmaceutically acceptable salt thereof and tadalafil or a pharmaceutically acceptable salt thereof as well as sucralose provided surprising benefits. Thus, it was observed that using glycerin in an amount of 0.1 to 20.0% of the chewing gum formulation according to the present invention surprisingly increased the viscosity of and provided a more soft chewing gum formulation. Additionally, based on its sweetening effect, using glycerin further helped in preventing the bitter taste of the formulation comprising the active agents such as dapoxetine and tadalafil which have unpleasant tastes. Sugar alcohols like glycerin are used as sweeteners since they affect the blood glucose and insulin levels to a less extent based on their slow absorption via the gastrointestinal tract. For this reason, the energy value of glycerin is lower than normal and was observed to have a synergistic effect together with sucralose in improving the taste of the chewing gum formulation. Additionally, glycerin prevents the microbial reproduction in the oral cavity based on its antimicrobial protection, and thus helps in preventing the tooth decays. In addition, glycerin which is also used as a plasticizer and softener provides an easy chewing of the chewing gum formulations.

According to another embodiment, keeping the ratio by weight of sucralose to glycerin in the formulation between 2:1 and 1:20, preferably between 1:1 and 1:10, provided a synergistic effect in improving the taste of the formulation.

According to another embodiment, the amount of dapoxetine or a pharmaceutically acceptable salt thereof is 5.0 to 30.0% by weight and the amount of tadalafil or a pharmaceutically acceptable salt thereof is 5.0 to 30.0% by weight in the chewing gum formulation according to the present invention.

Beside said active agents, sucralose, and glycerin, a chewing gum formulation according to the present invention further comprises at least one pharmaceutically acceptable excipient selected from a group consisting of other sweeteners, fillers, plasticizers, glidants, lubricants, antioxidants, flavoring agents, and colorants.

Beside sucralose, other sweeteners for use in the formulation according to the present invention include, but are not limited to acesulfame-K, aspartame, papain, tripolyphosphate, saccharine or saccharine sodium and calcium salts, sodium cyclamate, sucrose, fructose, glucose, or the mixtures thereof.

Suitable fillers for use in the formulation according to the present invention include, but are not limited to chewing gum bases, gum arabic, corn syrup, sucrose, inorganic salts, sorbitol, lactitol, erythritol, maltitol, mannitol, xylitol, isomalt, calcium salts, polysaccharides, dextrose, dicalcium phosphate, sodium chloride, sodium bicarbonate, dextrates, lactitol, maltodextrin, sucrose-maltodextrin mixtures, xylitol, trehalose, heavy magnesium carbonate, Pharmagum^{®}, Healthy in Gum^{®}, or the mixtures thereof.

Suitable plasticizers for use in the formulation according to the present invention include, but are not limited to candelilla wax, sodium stearate, sodium palmitate, triacetine, diethyl phthalate, dibutyl phthalate, tributyl citrate, or the mixtures thereof.

Suitable glidants for use in the formulation according to the present invention include, but are not limited to silicon dioxide, magnesium trisilicate, starch, talk, colloidal silicon dioxide, silicone hydrogel, or the mixtures thereof.

Suitable lubricants for use in the formulation according to the present invention include, but are not limited to magnesium stearate, sodium stearyl fumarate, polyethylene glycol, stearic acid, metal stearates, boric acid, sodium chloride benzoate and acetate, sodium lauryl sulfate, magnesium lauryl sulfate, or the mixtures thereof.

Suitable antioxidants for use in the formulation according to the present invention include, but are not limited to butyl hydroxy anisole, butyl hydroxy toluene, ascorbic acid, beta-carotene, alpha-tocopherol, propyl gallate, gentisic acid, sodium ascorbate, sodium bisulfate, sodium metabisulfate, monothioglycerol, cysteine, sodium thioglycolate, acetone sodium bisulfite, sodium bisulfate, sodium dithionite, gentisic acid ethanolamine, monosodium glutamate, sodium formaldehyde sulfoxylate, alpha tocopherol, or the mixtures thereof.

Suitable flavoring agents for use in the formulations according to the present invention include, but are not limited to fruit aromas such as orange, banana, strawberry, cherry, wild cherry, lemon, etc., and other aromas such as cardamom, anis, mint, menthol, vanillin, and the mixtures thereof. The flavoring agents assist in improving the taste of the chewing gum formulations together with sugar alcohols and the sweeteners.

Suitable colorants for use in the formulation according to the present invention include, but are not limited to, food, drug, and cosmetic (FD&C) dyes (e.g. FD&C blue, FD&C green, FD&C red, FD&C yellow, FD&C lake), ponceau, indigo drug & cosmetic (D&C) blue, indigotine FD&C blue, carmoisine indigotine (indigo carmine); iron oxides (e.g. iron oxide red, yellow, black), quinoline yellow, flame red, brilliant red (carmine), carmoisine, sunset yellow, etc., or the mixtures thereof.

The present invention is described in more details in the following examples. These examples are not limiting the scope of the present invention and should be considered under the light of the foregoing detailed disclosure.

### Examples

### Example 1 : Chewing gum formulation containing dapoxetine and tadalafil

| **Ingredients** | **Amount (%)** |
|---|---|
| Tadalafil | 5.00 - 30.00 |
| Dapoxetine | 5.00 - 30.00 |
| Gum base | 5.00 - 90.00 |
| Calcium carbonate | 5.00 - 60.00 |
| Sorbitol | 5.00 - 90.00 |
| Mannitol | 5.00 - 90.00 |
| Maltitol | 5.00 - 90.00 |
| Glycerin | 0.10 - 20.00 |
| Flavor | 0.10 - 5.00 |
| Gum arabic | 5.00 - 90.00 |
| Titanium dioxide | 0.005 - 2.00 |
| Candelilla wax | 0.10 - 20.00 |
| Sodium stearate | 0.10 - 2.00 |
| Tripolyphosphate | 0.10 - 2.00 |
| Sucralose | 0.10 - 2.00 |

### Production method:

The chewing gum base is heated until it is plasticized to an adequate extent. The plasticized gum base is mixed with calcium carbonate, sorbitol, mannitol, maltitol, gum arabic.Tadalafil, dapoxetine, glycerin, sucralose, titanium dioxide, candelilla wax, sodium stearate, tripolyphosphate and the flavor are stirred until a uniform mixture is obtained. The resulting mixture is rolled and cut into a desired size.

### Example 2: Chewing gum formulation containing dapoxetine and tadalafil

| **Ingredients** | **Amount (%)** |
|---|---|
| Tadalafil | 5.00 - 30.00 |
| Dapoxetine | 5.00 - 30.00 |
| Gum base | 5.00 - 90.00 |
| Sorbitol | 5.00 - 90.00 |
| Mannitol | 5.00 - 90.00 |
| Papain | 0.10 - 20.00 |
| Sucralose | 0.10 - 2.00 |
| Menthol powder | 0.10 - 2.00 |
| Liquid flavor | 0.01 - 2.00 |
| Isomalt | 5.00 - 90.00 |
| Glycerin | 0.10 - 20.00 |
| Talk | 0.10 - 10.00 |
| Flavor | 0.10 - 10.00 |

### Production method:

The chewing gum base is cooled down to -15°C, the resulting brittle mixture is sieved, disintegrated, and turned into a powder. The mixture is left standing at room temperature, then other excipients and the active agents of the formulation are added. The resulting powder mixture is granulated using a fluidized bed dryer. Then, the powder mixture of obtained granules is compressed in a tablet machine.

### Example 3: Chewing gum formulation containing dapoxetine and tadalafil

| **Ingredients** | **Amount (%)** |
|---|---|
| Tadalafil | 5.00 - 30.00 |
| Dapoxetine | 5.00 - 30.00 |
| Sugar | 5.00 - 90.00 |
| Gum base | 5.00 - 90.00 |
| Corn syrup | 0.50 - 60.00 |
| Glycerin | 0.10 - 20.00 |
| Flavor | 0.10 - 5.00 |
| Fructose | 0.10 - 5.00 |
| Sucralose | 0.10 - 2.00 |

### Production method:

The chewing gum base is heated until it is plasticized to an adequate extent. The plasticized chewing gum base is mixed with sugar. Tadalafil, dapoxetine, glycerin, sucralose, corn syrup, fructose, and the flavor are stirred until a uniform mixture is obtained. The resulting mixture is rolled and cut into a desired size.

### Example 4: Chewing gum formulation containing dapoxetine and tadalafil

| **Ingredients** | **Amount (%)** |
|---|---|
| Tadalafil | 5.00 - 30.00 |
| Dapoxetine | 5.00 - 30.00 |
| Gum base | 5.00 - 90.00 |
| Corn syrup | 0.50 - 60.00 |
| Glycerin | 0.10 - 20.00 |
| Flavor | 0.10 - 5.00 |
| Xylitol | 0.10 - 5.00 |
| Dextrose | 0.10 - 5.00 |
| Sucralose | 0.01 - 2.00 |
| BHT (butylated hydroxytoluene) | 0.001 - 2.00 |

### Production method:

The chewing gum base is cooled down to -15°C, the resulting brittle mixture is sieved, disintegrated, and turned into a powder. The mixture is left standing at room temperature, then other excipients and the active agents of the formulation are added. The resulting powder mixture is granulated using a fluidized bed dryer. Then, the powder mixture of obtained granules is compressed in a tablet machine.

### Example 5: Chewing gum formulation containing dapoxetine and tadalafil

| **Ingredients** | **Amount (%)** |
|---|---|
| Tadalafil | 5.00 - 30.00 |
| Dapoxetine | 5.00 - 30.00 |
| Gum base | 5.00 - 90.00 |
| Sorbitol | 5.00 - 90.00 |
| Mannitol | 5.00 - 90.00 |
| Glycerin | 0.10 - 20.00 |
| Flavor | 0.10 - 5.00 |
| Sodium bicarbonate | 5.00 - 60.00 |
| Sucralose | 0.10 - 2.00 |

### Production method:

The chewing gum base is heated until it is plasticized to an adequate extent. The plasticized chewing gum base is mixed with sorbitol and mannitol. Tadalafil, dapoxetine, glycerin, sodium bicarbonate, sucralose, and the flavor are stirred until a uniform mixture is obtained. The resulting mixture is rolled and cut into a desired size.

### Example 6: Chewing gum formulation containing dapoxetine and tadalafil

| **Ingredients** | **Amount (%)** |
|---|---|
| Tadalafil | 5.00 - 30.00 |
| Dapoxetine | 5.00 - 30.00 |
| Pharmagum^{®} | 5.00 - 90.00 |
| Sucralose | 0.10 - 2.00 |
| Glycerin | 0.10 - 20.00 |
| Flavor | 0.10 - 5.00 |
| Silicon dioxide | 0.10 - 2.00 |
| Sodium stearyl fumarate | 0.25 - 2.00 |

### Production method:

Pharmagum^{®} and other excipients, as well as the active agents are stirred until a uniform mixture is obtained. The resulting mixture is compressed on a conventional tablet machine.

## Claims

1. An oral chewing gum formulation comprising a combination of dapoxetine or a pharmaceutically acceptable salt thereof and tadalafil or a pharmaceutically acceptable salt thereof.

2. The chewing gum formulation according to claim 1, further comprising sucralose as a sweetener.

3. The chewing gum formulation according to claim 2, wherein the amount of sucralose is 0.10 to 2.00% by weight of the total formulation.

4. The chewing gum formulation according to claims 1 to 3, further comprising glycerin as an emulsifying agent.

5. The chewing gum formulation according to claim 4, wherein the amount of glycerin is 0.10 to 20.00% by weight of the total formulation.

6. The chewing gum formulation according to any of the preceding claims, wherein the ratio by weight of sucralose to glycerin is between 2:1 and 1:20.

7. The chewing gum formulation according claim 6, wherein the ratio by weight of sucralose to glycerin is between 1:1 and 1:10.

8. The chewing gum formulation according to claim 1, wherein the amount of dapoxetine or a pharmaceutically acceptable salt thereof is 5.0 to 30.0% by weight and the amount of tadalafil or a pharmaceutically acceptable salt thereof is 5.0 to 30.0% by weight.

9. The chewing gum formulation according to any of the preceding claims, further comprising at least one pharmaceutically acceptable excipient is selected from a group consisting of sweeteners, fillers, plasticizers, glidants, lubricants, antioxidants, flavoring agents, and colorants.

10. The chewing gum formulation according to claim 9, wherein beside sucralose the sweetener is selected from a group comprising acesulfame-K, aspartame, papain, tripolyphosphate, saccharine or saccharine sodium and calcium salts, sodium cyclamate, sucrose, fructose, glucose, or the mixtures thereof.

11. The chewing gum formulation according to claim 9, wherein the filler is selected from a group comprising gum bases, gum arabic, corn syrup, sucrose, inorganic salts, sorbitol, lactitol, erythritol, maltitol, mannitol, xylitol, isomalt, calcium salts, polysaccharides, dextrose, dicalcium phosphate, sodium chloride, sodium bicarbonate, dextrates, lactitol, maltodextrin, sucrose-maltodextrin mixtures, xylitol, trehalose, heavy magnesium carbonate, or mixtures thereof.

12. The chewing gum formulation according to claim 9, wherein the plasticizer is selected from a group comprising candelilla wax, sodium stearate, sodium palmitate, triacetine, diethyl phthalate, dibutyl phthalate, tributyl citrate, or the mixtures thereof.

13. The chewing gum formulation according to claim 9, wherein the glidant is selected from a group comprising silicon dioxide, magnesium trisilicate, starch, talk, colloidal silicon dioxide, silicone hydrogel, or the mixtures thereof.

14. The chewing gum formulation according to claim 9, wherein the lubricant is selected from a group comprising magnesium stearate, sodium stearyl fumarate, polyethylene glycol, stearic acid, metal stearates, boric acid, sodium chloride benzoate and acetate, sodium lauryl sulfate, magnesium lauryl sulfate, or the mixtures thereof.

15. The chewing gum formulation according to claim 9, wherein the antioxidant is selected from a group butyl hydroxy anisole, butyl hydroxy toluene, ascorbic acid, beta-carotene, alpha-tocopherol, propyl gallate, gentisic acid, sodium ascorbate, sodium bisulfate, sodium metabisulfate, monothioglycerol, cysteine, sodium thioglycolate, acetone sodium bisulfite, sodium bisulfate, sodium dithionite, gentisic acid ethanolamine, monosodium glutamate, sodium formaldehyde sulfoxylate, alpha tocopherol, or the mixtures thereof.
